# EUROPEAN PATENT SPECIFICATION

(11) **EP 4 650 502 B1**
(45) Date of publication and mention of the grant of the patent: **24.06.2026**
(21) Application number: 25175362.0
(22) Date of filing: 09.05.2025
(51) Int. Cl.: D04H 1/407, D04H 1/4391, D04H 1/56, D04H 3/16, D04H 3/018, B32B 5/26, B32B 5/30, D01D 4/02, D01D 5/08, D01D 5/253, A61F 13/15, A61F 13/53, A61F 13/534

(54) **COMPOSITE ABSORBENT TAPE**
ZUSAMMENGESETZTES ABSORBIERENDES BAND
BANDE ABSORBANTE COMPOSITE

(30) Priority: 13.05.2024 IT 202400010729
(43) Date of publication of application: 19.11.2025
(73) Proprietor: Fratelli Ceccato Milano S.r.l., 20123 Milano (IT)
(72) Inventor: ANGELICO, Giuseppe, 20095 Cusano Milanino (IT)
(74) Representative: Lunati & Mazzoni S.r.L.

(56) References cited:
- WO-A1-2020/103964
- WO-A1-2021/089731
- WO-A1-2021/198894
- WO-A1-2021/228291
- WO-A1-2022/120693
- US-A1- 2015 322 592

## Description

The object of the present invention is a composite absorbent tape of the type as defined in the first claim.

In particular, the present invention relates to a tape in which particles mixed with continuous microfilaments are positioned within the pores of a high thickness tape and a process for the formation of such tapes, which can be applied in filtration or, especially if the particles are liquid-absorbent, in hygienic absorbent articles.

As is known, tapes comprising particulate materials are widely used, for example, without limitations, in air purification, where the particles remove contaminants with an adsorption mechanism. Another widespread use involves absorbent structures for absorbent articles, for example for personal hygiene, such as disposable diapers for children, training pants for children or underwear for adult incontinence, which are designed to absorb and contain body exudates, especially urine.

These absorbent articles comprise several layers which perform different functions, typically including a topsheet, a backsheet and, among other layers, an absorbent core. The absorbent core must be able to absorb and retain liquid exudates for a prolonged period of time, such as overnight for a diaper, should minimize re-wetting to keep the wearer dry, and avoid soiling clothing or sheets. Modern absorbent cores typically comprise absorbent structures composed of super-absorbent polymer (SAP) particles, also called absorbent gelling materials (AGM), and fibrous materials, which may be natural, such as cellulose fibres, modified natural, such as regenerated cellulose-based materials, or synthetic fibres.

It is well known that absorbent structures can be formed "in-line" or "in-situ" on a processing line to form the complete absorbent article, see, for example WO2022/120693A1, which describes an absorbent core for use in an absorbent article, comprising a liquid-permeable top cover layer, a bottom cover layer and a central layer with a high fill coefficient between the top and bottom cover layers, and first and second super-absorbent polymers which penetrate at least partially into the high-fill-coefficient central layer, in addition to adhesively-fixed cover layers. Furthermore, WO2014/001487 discloses particles embedded in a porous fabric and ultrasonically immobilized between the cover layers.

However, these approaches require that every line for the production of articles, also known as a converter, be equipped with appropriate handling systems for adding particles, as well as unwinding and joining systems for the preformed tapes. Furthermore, the formation of the absorbent core may limit the overall production volume.

As an alternative to forming the core in-line, composite absorbent tapes comprising particles, such as super-absorbents, may be formed off-line at high production volumes, which may be delivered as so-called roll stocks to the conversion lines for forming articles to be packaged and/or combined with other elements to form the absorbent articles, thereby advantageously simplifying the conversion equipment and process and offering production cost advantages due to the high production volumes.

WO2021/188330A1 discloses an absorbent core for use in an absorbent article, comprising a liquid permeable top layer, a bottom layer, a high thickness central layer and super-absorbent polymer particles at least partially distributed in the central layer.

WO2020/025401 discloses an absorbent core comprising at least a top layer and a bottom layer, each of which is composed of 0 to 10 w% of fibrous material (natural or synthetic) and 90 to 100 w% of absorbent polymer particles. The absorbent core is made by dropping a first absorbent polymer or a mixture on one side of a nonwoven material. The composite is then rotated and the second water-absorbent polymer is dropped onto the other side of the nonwoven fabric, which is also covered by a lower fabric. Then, the absorbent composite is cut to the desired width and wrapped.

However, better approaches regarding the structure are still needed, such as avoiding the use of glues, so as to facilitate the recycling process of factory and post-consumer waste and reduce the ecological footprint of such products. Furthermore, the application of glues involves considerable energy consumption and the handling of liquids may be adversely affected by the use of adhesives in the absorbent structures.

Approaches that avoid the use of glues are also known, as for example in the case described by WO2013/152809, where the particles are incorporated into the pores of a preformed tape. For satisfactory particle containment, such loaded tapes are wrapped in a separate step in coating materials, such as fabrics or nonwoven tapes.

WO2020/103964A1 discloses an open, porous spunbond tape of continuous crimped fibres, wherein at least a portion of the pores may be filled with particles, such as super-absorbents. Even when using crimped fibres, which result in a reduction in production speed, their thickness results in poor entrapment, requiring additional tape wrapping, and relatively low softness.

WO2021/198894 instead describes a tape comprising meltblown elastomeric fibres, optionally also meltblown non-elastomeric filaments, with particulate material trapped within the pores of the tape and at least in part adhering to the fibres due to the adhesiveness of the latter upon meltblowing. Optionally, such a composite can be combined with auxiliary tapes, such as nonwoven fabrics, to retain particles or high-density materials for better distribution of liquids during use.

This approach involves the use of non-continuous meltblown fibres, formed with "air-knife" technology, wherein the polymer filaments are contacted at the exit of the forming nozzle with attenuating air from an angled direction, thereby cutting the filaments into short pieces, which have reduced particle containment functionality. Furthermore, the need to use elastomeric polymers increases costs and reduces production flexibility and speed, which may not be acceptable.

In general, although off-line forming of absorbent structures offers advantages over in-line forming, such as a very high production volume with a single production unit, there is still a need to improve the economics and/or properties of the resulting absorbent structures. Furthermore, the use of adhesives in the absorbent structure complicates recycling, in particular that of factory waste, and can have a negative impact on consumers' perception as an unnecessary chemical.

WO2021/089731 A1 discloses a co-mingled nonwoven web obtained by combining coaxially meltblown fibres with a stream of particulate or short fibre material and then depositing the resulting co-mingled mixture onto a collector.

In this situation, the technical task underlying the present invention is to devise a composite tape that can substantially obviate at least part of the above-mentioned drawbacks.

Within the scope of said technical task it is an important object of the invention to obtain a composite tape, and related manufacturing method, which is economically feasible.

In addition, another important object of the invention is the manufacture of a composite tape, and a related manufacturing method, which makes it possible to obtain a high production volume with a production unit and less expensive raw materials, in particular without adhesives.

In conclusion, a further object of the invention is to obtain a composite tape which, in view of the advantages just described, is very efficient without compromising the containment of the particles before and during production, but also during use. The technical task and the specified objects are achieved by a composite tape as defined in claim 1. Further aspects of the invention relate to a plant for manufacturing the tape, as defined in claim 10, and a method for manufacturing the tape, as defined in claim 12.

Preferred embodiments are highlighted in the dependent claims.

The features and advantages of the invention are clarified below by the detailed description of preferred embodiments of the invention, with reference to the attached drawings, in which:

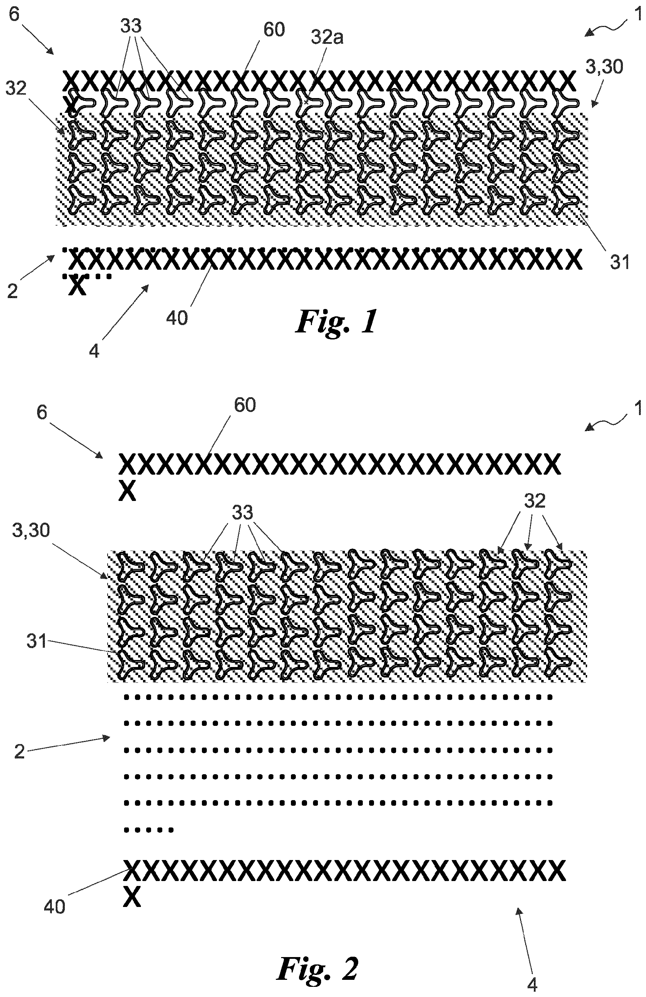

Fig. 1 shows a schematic view of the layers of a first embodiment of composite absorbent tape according to the invention including a matrix and in which each first polymer filament defines a same outline;
Fig. 2 illustrates an exploded view of the absorbent composite tape of Fig. 1;

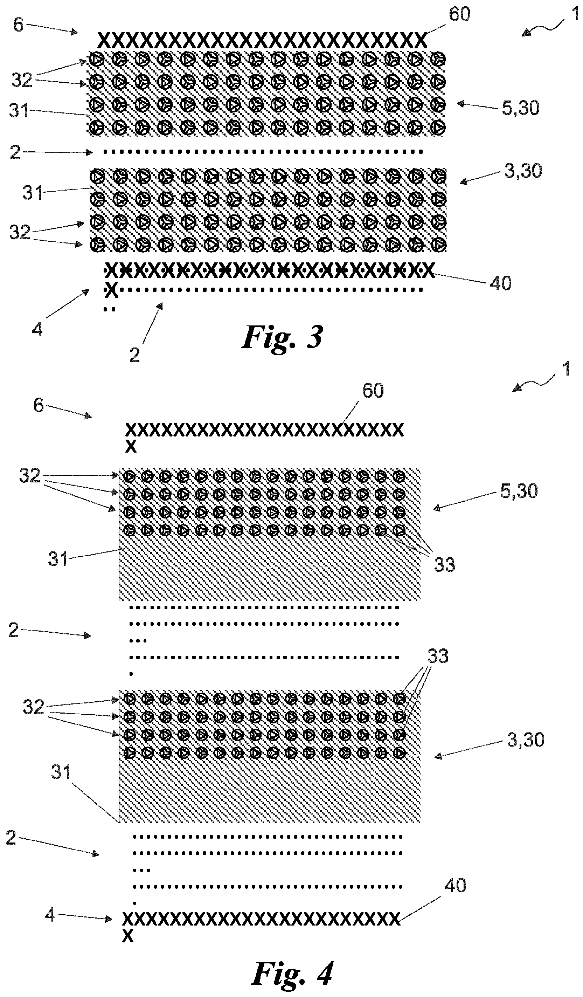

Fig. 3 a schematic view of the layers of a second embodiment of composite absorbent tape according to the invention including a matrix and in which the first polymer filaments define different and distinct outlines and in which a third and a fourth layer are also present;
Fig. 4 shows an exploded view of the absorbent composite tape of Fig. 3;
Fig. 5 shows a tube of the first spinneret of a plant for manufacturing a tape according to the invention in which the outer surface is shaped like the inner surface;
Fig. 6a illustrates a sectional view in the section plane of an outline having a first concave shape of a tube of the first spinneret for a plant for manufacturing a tape according to the invention wherein a convex portion is shown highlighted in hatching;
Fig. 6b is a sectional view on the section plane of an outline having a second concave shape of a tube of the first spinneret for a plant for manufacturing a tape according to the invention wherein a convex portion is shown highlighted in hatching;
Fig. 6c represents a sectional view on the section plane of an outline having a third concave shape of a tube of the first spinneret of a plant for manufacturing a tape according to the invention wherein a convex portion is shown highlighted in hatching;
Fig. 6d shows a sectional view on the section plane of an outline having a fourth convex shape of a tube of the first spinneret of a plant for manufacturing a tape according to the invention;
Fig. 6e illustrates a sectional view on the section plane of an outline having a fifth convex shape of a tube of the first spinneret of a plant for manufacturing a tape according to the invention;
Fig. 6f is a sectional view on the section plane of an outline having a sixth concave shape of a tube of the first spinneret for a plant for manufacturing a tape according to the invention wherein a convex portion is shown highlighted in hatching;
Fig. 6g represents a sectional view on the section plane of an outline having a seventh concave shape of a tube of the first spinneret for a plant for manufacturing a tape according to the invention wherein a convex portion is shown highlighted in hatching;
Fig. 6h shows a sectional view on the section plane of an outline having a eight concave shape of a tube of the first spinneret for a plant for manufacturing a tape according to the invention wherein a convex portion is shown highlighted in hatching;
Fig. 6i illustrates a sectional view on the section plane of an outline having a ninth concave shape of a tube of the first spinneret for a plant for manufacturing a tape according to the invention wherein a convex portion is shown highlighted in hatching;
Fig. 6j is a sectional view on the section plane of an outline having a tenth concave shape of a tube of the first spinneret for a plant for manufacturing a tape according to the invention wherein a convex portion is shown highlighted in hatching;
Fig. 7a represents a perspective view of a tube of the first spinneret for a plant for manufacturing a tape according to the invention wherein the outer surface has a cylindrical shape and the outline has the shape of Fig. 6a;
Fig. 7b shows a perspective view of a tube of the first spinneret for a plant for manufacturing a tape according to the invention wherein the outer surface has a cylindrical shape and the outline has the shape of Fig. 6b;
Fig. 7c illustrates a perspective view of a tube of the first spinneret for a plant for manufacturing a tape according to the invention wherein the outer surface has a cylindrical shape and the outline has the shape of Fig. 6c;
Fig. 7d is a perspective view of a tube of the first spinneret for a plant for manufacturing a tape according to the invention wherein the outer surface has a cylindrical shape and the outline has the shape of Fig. 6d;
Fig. 7e represents a perspective view of a tube of the first spinneret for a plant for manufacturing a tape according to the invention wherein the outer surface has a cylindrical shape and the outline has the shape of Fig. 6e;
Fig. 7f shows a perspective view of a tube of the first spinneret for a plant for manufacturing a tape according to the invention wherein the outer surface has a cylindrical shape and the outline has the shape of Fig. 6f;
Fig. 7g illustrates a perspective view of a tube of the first spinneret for a plant for manufacturing a tape according to the invention wherein the outer surface has a cylindrical shape and the outline has the shape of Fig. 6g;
Fig. 7h is a perspective view of a tube of the first spinneret for a plant for manufacturing a tape according to the invention wherein the outer surface has a cylindrical shape and the outline has the shape of Fig. 6h;
Fig. 7i represents a perspective view of a tube of the first spinneret for a plant for manufacturing a tape according to the invention wherein the outer surface has a cylindrical shape and the outline has the shape of Fig. 6i;
Fig. 7j shows a perspective view of a tube of the first spinneret for a plant for manufacturing a tape according to the invention wherein the outer surface has a cylindrical shape and the outline has the shape of Fig. 6j;
Fig. 8a illustrates a cross-sectional view of a first spinneret of a plant for manufacturing a tape according to the invention including a plurality of tubes wherein the outer surface is outlined like the inner surface and wherein the tubes have the same outline on the same column and outlines alternating on the same row;
Fig. 8b is a cross-sectional view of a first spinneret of a plant for manufacturing a tape according to the invention including a plurality of tubes wherein the outer surface is outlined like the inner surface and wherein the tubes have the same outline;
Fig. 8c represents a cross-sectional view of a first spinneret of a plant for manufacturing a tape according to the invention including a plurality of tubes mutually stacked and alternating in columns of tubes each defining a circular outline and wherein the outer surface is outlined like the inner surface;
Fig. 8d shows a cross-sectional view of a first spinneret of a plant for manufacturing a tape according to the invention including a plurality of tubes wherein the outer surface is outlined like the inner surface and wherein the tubes have the same outline on the same column and outlines respectively concave and convex alternating on the same row;
Fig. 9a illustrates a cross-sectional view of a first spinneret of a plant for manufacturing a tape according to the invention including a plurality of tubes wherein the outer surface is cylindrical and wherein the tubes have the same concave outline;
Fig. 9b is a cross-sectional view of a first spinneret of a plant for manufacturing a tape according to the invention including a plurality of tubes wherein the outer surface is cylindrical and wherein the tubes have the same convex triangular outline;
Fig. 9c represents a cross-sectional view of a first spinneret of a plant for manufacturing a tape according to the invention including a plurality of tubes wherein the outer surface is cylindrical and wherein the tubes have the same convex, nearly rectangular outline;
Fig. 9d shows a cross-sectional view of a first spinneret of a plant for manufacturing a tape according to the invention including a plurality of tubes wherein the outer surface is cylindrical and wherein the tubes have the same concave, nearly star-shaped outline;

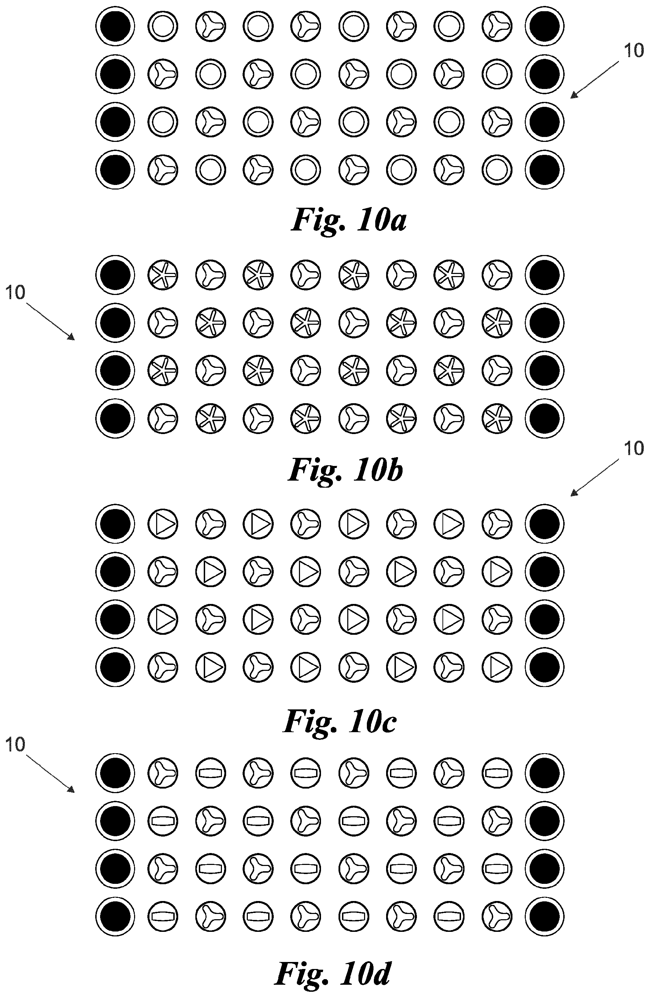

Fig. 10a illustrates a cross-sectional view of a first spinneret of a plant for manufacturing a tape according to the invention including a plurality of tubes wherein the outer surface is cylindrical and wherein the tubes have the same concave outline and wherein the tubes are alternating in a chequered pattern with tubes having a circular outline;
Fig. 10b is a cross-sectional view of a first spinneret of a plant for manufacturing a tape according to the invention including a plurality of tubes wherein the outer surface is cylindrical and wherein the tubes have two different concave outlines and wherein the tubes with different outlines are alternating in a chequered pattern;
Fig. 10c represents a cross-sectional view of a first spinneret of a plant for manufacturing a tape according to the invention including a plurality of tubes wherein the outer surface is cylindrical, the tubes have two different outlines respectively concave and convex and wherein the differently outlined tubes are alternating in a chequered pattern;
Fig. 10d shows a cross-sectional view of a first spinneret of a plant for manufacturing a tape according to the invention including a plurality of tubes wherein the outer surface is cylindrical, the tubes have two different outlines respectively concave and convex and wherein the differently outlined tubes are alternating in a chequered pattern;
Fig. 11 shows a schematic side view of a plant for manufacturing a tape according to the invention including an unwinding unit for the deposition of the matrix;
Fig. 12 illustrates a schematic view of a plant for manufacturing a tape according to the invention wherein the sequence of the steps of a first embodiment of the method for manufacturing a tape according to the invention is shown;
Fig. 13 is a schematic view of a plant for manufacturing a tape according to the invention wherein the sequence of the steps of a second embodiment of the method for manufacturing a tape according to the invention is shown;
Fig. 14 represents a schematic view of a plant for manufacturing a tape according to the invention wherein the sequence of the steps of a third embodiment of the method for manufacturing a tape according to the invention is shown;
Fig. 15 shows a schematic side view of a first embodiment of a plant for manufacturing a tape according to the invention wherein the unwinding unit for the deposition of the matrix is absent; the plant is intended for manufacturing tapes of the wipes type;
Fig. 16 illustrates a schematic view of the plant in Fig. 15 in a further embodiment wherein the filaments are distributed on the inclined conveyor;
Fig. 17 is a schematic side view of a third embodiment of a plant for manufacturing a tape according to the invention wherein the unwinding unit for the deposition of the matrix is absent; the plant is intended for manufacturing tapes of the wipes type;
Fig. 18 represents a schematic side view of a fourth embodiment of a plant for manufacturing a tape according to the invention wherein the unwinding unit for the deposition of the matrix is absent; the plant is intended for manufacturing tapes of the wipes type;
Fig. 19 shows a schematic side view of a fifth embodiment of a plant for manufacturing a tape according to the invention wherein the unwinding unit for the deposition of the matrix is absent; the plant is intended for manufacturing tapes of the wipes type.

In this document, when measurements, values, shapes, and geometric references (such as perpendicularity and parallelism) are associated with words like "approximately" or other similar terms, such as "almost" or "substantially", they are to be understood as excluding measurement errors or inaccuracies due to production and/or manufacturing errors and, above all, as having less than a slight deviation from the associated value, measurement, shape, or geometric reference. For example, if associated with a value, such terms preferably indicate a deviation by no more than 10% of the value itself.

Moreover, when used, terms such as "first", "second", "upper", "lower", "main" and "secondary" do not necessarily identify an order, a priority of relationship or a relative position, but can simply be used to clearly distinguish between their different components.

Unless otherwise specified, as reflected in the following discussions, terms such as "processing", "computing", "determination", "calculation", or the like are considered to refer to the action and/or processes of a computer or similar electronic computing device that manipulates and/or transforms data represented as physical, such as electronic quantities of records of a computer plant and/or memories, into other data similarly represented as physical quantities within computer plants, records, or other information storage, transmission, or display devices.

Unless otherwise stated, the measurements and data reported in this text shall be considered as provided in International Standard Atmosphere ICAO (ISO 2533:1975).

With reference to the Figures, the composite tape according to the invention is globally referred to as number 1.

In general terms, the term "fibre" herein refers to elongated fibres having a length/diameter ratio of at least about 3:1, often greater than 10:1, or even greater than 100:1. Synthetic fibres or artificial fibres based on natural materials are typically formed by the solidification of continuous filaments of molten polymers, which may be homogeneous or monocomponent polymers, or mixtures of polymers, or which may form distinct transverse regions within a filament, with the possibility of creating crimped or crimpable fibres. The term "filament" is also used interchangeably for essentially continuous solidified fibres, while "fibres" is used to describe non-continuous structures. A typical process for forming a tape from essentially continuous filaments is known as "spunbonding", see for example US5935512, which creates essentially continuous filaments with a diameter of between about 1 and 50 µm, often between 15 and 35 µm.

"Microfibres" are used to describe fibres obtained by the formation of meltblown tapes, as known, for example, from US8017534, in which low viscosity polymers are extruded through a nozzle and attenuated by a high speed air flow blown at an angle to the formed filament. In this way the melt disperses, solidifies and breaks into a fibrous tape.

More recently, CAM (co-axially meltblowing) technology has gained particular interest, providing essentially continuous microfilaments. The formation of CAM filaments is described in more detail, for example, in US9303334 and represents an important element for the present invention, as will be discussed in further detail below.

Although the polymers for these filaments can be chosen from a wide range, the preferred polymers are polyolefins and, even more preferred, polypropylene. Elastomeric polyolefins can be used, but this is not necessary from a performance point of view and preferably not from a commercial point of view due to their higher cost. If one is employed, it should be at a level of less than 5 w%, based on the total weight of the filament polymer. The polymers preferably exhibit a melt flow index MFR of 25 MFR of greater than about 25 g/10 min, preferably greater than about 45 g/10 min, typically less than about 2000 g/10 min, as can be determined by ASTM D1238 and ISO 1133, and for polypropylene as a polymer that can be suitably processed with current equipment and processes, is expressed in gram units for 10 minutes at 210°C and 2.16 kg load.

In the present context, a "tape" comprises a matrix of fibres or filaments of a single type, or of a mixture, which may be directionally or randomly oriented, and bonded by friction, and/or adhesion, and/or cohesion, where the latter may be imparted directly after the formation of the filaments, for example in the step of laying the fibre or filament. Typically, a tape is self-supporting and allows handling on production or processing plants, even though a single sub-thread of a composite tape may not have sufficient integrity on its own. A tape may comprise particles such as a mixture of filaments and particles. A "composite tape" refers to a combination of (sub)tapes in a layered configuration, while a "continuous tape" is essentially infinite in length or in the x-direction corresponding, during production, to the machine direction (MD), and may be wound onto rolls or reels, or "streamered" into boxes, which may be joined together to form the essentially infinite tape. Thus, a continuous tape has a width perpendicular to the direction of the length, corresponding to the transverse direction during production, which may extend for several meters during the production of the tape, or for less than one meter and corresponding to the width used in the processing or forming of the articles, and a thickness, perpendicular to the length and the width, and significantly smaller than one of the two.

Another element important to the present invention is a fabric with high thickness. The term "high loft" refers to voluminous, low-density fabrics, as compared to flat, paper-like fabrics. High loft fabrics are characterized by a relatively high porosity. This means that there is a relatively large amount of void space between the fibres in which particles, such as those of super-absorbent polymer, can be distributed. The high loft web (without super-absorbent particles) suitable for the present invention may have a pressure density of 0.83 kPa (0.12 psi) of less than 0.15 g/cm³, particularly between 0.01 g/cm³ and 0.15 g/cm³, or between 0.05 g/cm³ and 0.12 g/cm³, or between 0.08 g/cm³ and 0.10 g/cm³. Preferably, the high thickness web maintains its opening even at a pressure greater than 4.14 kPa (0.6 psi) with a density of less than about 0.20 g/cm³, particularly between 0.01 g/cm³ and 0.20 g/cm³, or between 0.05 g/cm³ and 0.15 g/cm³³, where the density may be calculated by dividing the basis weight of the high loft layer by its thickness measured at the respective pressure as indicated. The basis weight and thickness of a suitable high loft may be adapted to the specificities of the particular application. In particular, the high loft (sub)web may have a thickness of at least 0.30 mm, in particular between 0.30 mm and 2.00 mm, or between 0.50 mm and 1.5 mm, measured at a pressure of 4.14 kPa (0.6 psi). The basis weight of the high thickness (sub)web can vary from 15 g/m² to 500 g/m², in particular from 30 g/m² to 200 g/m², for example from 50 g/m² to 120 g/m². The fibres forming the high thickness web may be constituted partially or entirely of relatively resilient synthetic fibres, in particular fibres of polypropylene (PP), polyamide (PA, such as nylons) or polyethylene terephthalate (PET). The diameter of the fibres can range, for example, from 0.01 mm to 0.50 mm. A particular exemplary embodiment of a high volume fabric may be an air bonded carded tape made of staple fibres that are sent through a combing or carding unit, which generally separates and aligns the staple fibres in the machine direction to form a fibrous nonwoven tape generally oriented to the machine direction. This tape is then pulled through a heated drum, creating bonds throughout the fabric without applying specific pressure (air bonding process).

Optionally, the high flow web may comprise secondary layers having different properties and functionalities. Such properties may be density, fibre thickness, or fibre composition, and the difference in property should be greater than about 3%, or about 5, or even about 10%, based on the respective highest value.

A third element important to the present invention are particles that are positioned within the pores of the high thickness web.

Such particles may provide a wide range of functionality, such as coloring, washing, or adsorbing gases or gaseous contaminants for filtration purposes. A particular application concerns the absorption of liquids, whereby the particles are suitable for absorbing liquids, such as water or aqueous solutions, such as body exudates, at multiples of their own weight. "Superabsorbent polymers" (SAP) means absorbent materials capable of absorbing at least 10 times their weight of an aqueous 0.9% saline solution as measured by the Centrifuge Retention Capacity (CRC) test (EDANA method NWSP 241.0.R2 (19)). SAPs preferably have a CRC value of at least 15 g/g. SAPs are typically cross-linked polymers that are insoluble in water but can absorb large amounts of fluids. SAPs are in particulate form so as to be flowable in the dry state. Typical particulate SAPs are polyacrylated polymers, but it is not excluded that other polymeric materials may also be used. For example, starch-based particulate absorbent polymer materials, polyacrylamide copolymer, ethylene maleic anhydride copolymer, cross-linked carboxymethylcellulose, polyvinyl alcohol copolymers, cross-linked polyethylene oxide, and starch-grafted polyacrylonitrile copolymer can be used.

SAPs may be polyacrylates and polyacrylic acid polymers cross-linked internally and/or at the surface. The superabsorbent polymer of the invention may be selected from internally and surface cross-linked polyacrylates and polyacrylic acid polymers. The superabsorbent polymers can be internally cross-linked, i.e. the polymerization takes place in the presence of compounds with two or more polymerizable groups that can be free-radically copolymerized in the polymer network. Preferably, the SAP particles comprise cross-linked polymers of polyacrylic acids or their salts or polyacrylates or their derivatives.

Particles may be relatively small (less than 1 mm in their longest dimension) in the dry state and may have an approximately circular shape, but granules, fibres, flakes, spheres, powders, platelets, and other shapes are known to those skilled in the art. Spherical shaped particles can facilitate penetration into the pores of a high density tape.

In the composite tape 1 according to the present invention, the particles are mixed with the polymer microfilaments, thereby forming a filament-particle mixture. This is readily understood when considering the manner in which such a mixture may be produced, i.e. by introducing a stream of particles into the attenuation zone of a filament forming apparatus, as will be discussed in more detail below. As such, the filaments entangle the particles as they collectively travel toward a collection tape. While the open porous tape with high thickness is already positioned on the collection tape, the mixture is deposited on the high pile tape and at least a part of the mixture of filaments and particles penetrates into the pores of the high pile tape, possibly with the support of a step that promotes penetration, such as vibration or air aspiration.

After having set forth the foregoing premises, in detail, the tape 1 comprises at least one or more matrix 2, so that the tape 1 forms an absorbent fabric, particularly suitable for use in manufacturing diapers.

The matrix 2 is preferably made of high thickness polymeric fibres. In addition, the matrix 2 comprises pores, as better explained below.

The tape 1 comprises, in each case, at least a first layer 3.

The first layer 3 is in contact with the matrix 2. In addition, the first layer 3 includes a mixture 30.

The mixture 30 at least in part penetrates into the pores. Thus, the mixture 30 comprises, mutually mixed together, at least a plurality of particles 31 and a plurality of first polymer filaments 32.

The particles 31 are preferably made of cellulose.

The first polymer filaments 32 are preferably continuous. Furthermore, the first polymer filaments 32 define, before being mixed with the particles 31, a development axis 32a and, at least in part, a plurality of mutually identical outlines 33 arranged in succession along the development axis 32a. The terms "at least in part" mean that not all the first polymer filaments 32 must necessarily define the outlines 33 along their development axis 32a.

The tape 1 therefore comprises at least one second layer 4.

The second layer 4 is preferably made of second polymer filaments 40.

The second polymer filaments 40 are preferably continuous. Further, the second polymer filaments 40 form a surface layer of the tape 1. Thus, the second layer is brought into contact with the matrix 2, and/or the first layer 3 without penetrating the pores.

The tape 1 may, therefore, for example comprise a sandwich structure in which there is a second layer 4 on which a matrix 2 is superimposed and therefore a first layer 3 superimposed on the matrix 2 or comprised between the second layer 4 and the matrix 2.

Or, the tape 1 may also further comprise a fourth layer 6.

If present, the fourth layer includes third continuous polymer filaments 60.

Furthermore, similarly to the second third polymer filaments 40, the third polymer filaments 60 also form a surface layer of the tape 1. Then, the fourth layer 6 can be brought into contact with the matrix 2 and/or the first layer 3 without penetrating the pores. Preferably, if the fourth layer 6 is present, it is placed at an opposite side to the second layer 4.

Then, the tape 1 may comprise a sandwich formed, in order, by second layer 4, first layer 3 or matrix 2, matrix 2 or first layer 3, and fourth layer 6.

Additionally or alternatively, the tape 1 may also comprise a third layer 5.

If present, the third layer 5 is in contact with the matrix 2 at a side opposite to the first layer 3.

Thus, advantageously, the third layer 5 also includes a mixture 30 at least partly penetrating the pores.

Thus, if the third layer 5 is present and the fourth layer 6 is also present, the latter can also be contacted with the third layer 5 without penetrating the pores.

The tape 1 could therefore include a sandwich formed, in the order, of second layer 4, first layer 3, matrix 2, third layer 5, and fourth layer 6.

There could also be two matrices 2, one for each layer 3, 5. Then, the sandwich could be formed in order, by second layer 4, matrix 2, first layer 3, matrix 2, third layer 5, and fourth layer 6.

It is important to note that, according to the invention, even at least part of the second polymer filaments 40 and/or the third polymer filaments 60 could define a respective development axis 32a and respective outlines 33 similarly to the first polymer filaments 32.

In any case, advantageously, the outline 33 is determined on a section plane 32b. The section plane 32b is preferably normal to the development axis 32a. Therefore, the section plane 32b is substantially a virtual plane which, by cutting the polymer filament 32, 40, 60 normally to the development axis 32a, defines on itself the outline 33.

The outline 33 also defines a first extension area. The extension area is the portion of two-dimensional space contained within the outline 33.

Thus, the outline 33 is preferably inscribable in a circle. The circle is also determined on the section plane 32b. Of course, the circle is trivially a virtual geometric element within which the outline 33 is geometrically inscribable.

The circle, in addition, itself defines a second extension area on the section plane 32b. Hence, the second area of extension being determined by the two-dimensional space contained within the circle, it can be as known obtained by the formula A=π*r². Advantageously, the outline 33 does not have a shape corresponding to the circle. In fact, advantageously, the first extension area is less than 90% of the second extension area. Even more in detail, preferably, the first extension area is less than 60% of the second extension area.

Thus, the outline 33 can be made according to different embodiments.

For example, the outline 33 may be a convex figure. As is known, a convex figure is a figure in which any segment joining any two points of it is entirely contained in the figure itself.

Therefore, if the outline 33 is convex, it preferably defines a first dimension **33a** and a second dimension **33b.**

The first dimension 33a is substantially the maximum dimension that the outline 33 determines in one direction. The second dimension 33b is also the maximum dimension in a direction perpendicular to the first dimension 33a.

Preferably, the second dimension 33b is less than 90% of the first dimension 3a. Even more in detail, the second dimension 33b may be less than 60% of the first dimension 33a.

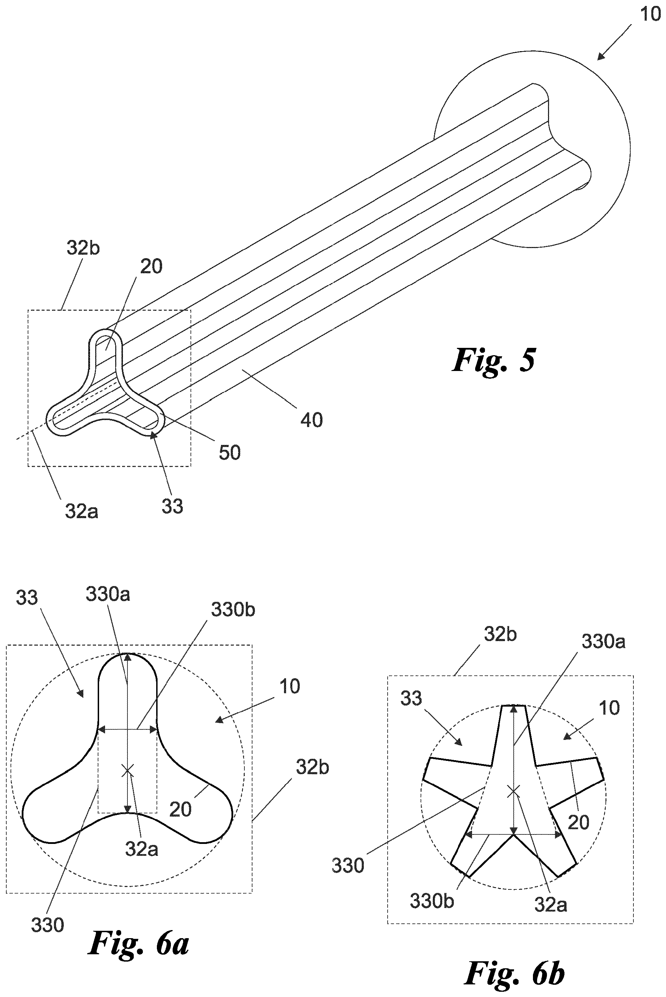

In addition, the dimensions may refer to geometrically well-defined outlines 33. For example, a convex outline 33 may have a nearly equilateral triangular shape, as shown in Fig. 5d, or almost rectangular, possibly even slightly rounded on the sides, as shown in Fig. 5e.

Of course, in the case of a triangle, the first dimension 33a may be provided by the height, while the second dimension 33b may be provided by the base side on which the height lies. In the case of the rectangle, the dimensions 33a, 33b may correspond to the respective sides.

In other embodiments, the outline 33 may instead be concave. Dually with respect to convexity, the concavity of a figure is manifested when there is at least one segment that joins a pair of points of the figure does not belong entirely to the figure itself.

Thus, if the outline 33 is concave, it preferably includes at least one convex portion **330.** The convex portion 330 is a part of a concave outline 33 that can be identified within the outline 33, in such a way as to be delimited at least in part, and which has the characteristic of convexity.

Therefore, also the convex portion 330 can, similarly to the convex outline 33, define a third dimension **330a** and a fourth dimension **330b.**

The third dimension 330a is substantially the maximum dimension that the convex portion 330 determines in one direction. The fourth dimension 330b is also the maximum dimension in a direction perpendicular to the third dimension 330a.

Preferably, the fourth dimension 330b is less than 90% of the third dimension 330a. Even more in detail, the fourth dimension 330b may be less than 60% of the third dimension 330a.

As previously, dimensions may refer to geometrically well-defined convex portions 330. For example, a convex portion 33 of an outline 33 may have an almost triangular shape, as shown for example in Fig. 5h, or almost rectangular as shown in Figs. 5g and 5j, possibly with a bevelled side as in Fig. 5a, or even trapezoidal, as shown in Figs. 5b-5c.

Of course, in the case of a triangle, the first dimension 33a may be provided by the height, while the second dimension 33b may be provided by the base side on which the height lies. In the case of the rectangle, the dimensions 33a, 33b may correspond to the respective sides.

More generally, a concave outline 33 may be formed by two or more mutually crossed convex portions 30. Hence, the concave outline 33 may define a cross shape having three to five points. For example three, as shown in Figs. 5a and 5c, or four as in Figs. 5f and 5i, or even five as in Figs. 5b and 5j.

The invention also makes it possible to manufacture a new plant 100 for manufacturing a composite tape.

Suitably, the new plant 100 allows the tape 1 to be manufactured as previously described.

Thus, the plant 100 comprises, at least, a conveyor 101.

The conveyor 101 defines at least one support surface 101a. The support surface 101a is substantially the surface on which the tape 1 can be made, for example by deposition.

The conveyor 101 may, for example, comprise a conveyor belt sliding along a closed path and guided by winders.

In any case, the plant 100 preferably comprises at least one first coaxial blowing unit 102.

The first coaxial blowing unit 102 is adapted to allow the deposition of the second layer 4 on the conveyor 101.

In addition, the plant 100 also comprises at least one unwinding unit 103.

The unwinding unit 103 is adapted to allow the deposition of the matrix 2 on the second layer 4.

The plant 100 also comprises at least one first mixing unit 104.

The first mixing unit 104 is adapted to manufacture the first layer 3 to allow its deposition on the matrix 2.

Then, the first mixing unit 104, preferably including a first supply unit 1040 adapted to produce the particles 31, a first polymerization unit 1041 adapted to produce the first polymer filaments 32, and a first spinneret 1042 adapted to outline the first polymer filaments 32, before conveying them towards the particles 31 to mix them to manufacture the mixture 30.

Advantageously, the spinneret 1042 comprises a plurality of tubes 10.

The tubes 10 develop, of course, along a respective development axis 32a.

At least part of the tubes 10, therefore, defines an outline 33 determined on the section plane 32b, i.e. an outline corresponding to that of the filaments, in particular the first polymer filaments 32, that exit from the tubes 10.

Thus, by analogy with the polymer filaments 32, 40, 60 described above, the tube 10 is, as such, a substantially elongated element comprising a cavity through which liquid polymer can percolate to allow extrusion, e.g. from a spinneret.

The tube 10, therefore, defines the development axis 32a.

The development axis 32a is substantially the axis about which the tube 10 develops. Furthermore, the development axis 32a is the axis along which the polymeric liquid can flow and, therefore, is the axis along which the cavity defined by the tube 10 develops.

The tube 10, therefore, comprises at least one inner surface 20.

The inner surface 20 is substantially closed. Furthermore, it develops around the development axis 32a, since it is in fact facing onto it.

The inner surface 20, therefore, encloses the cavity.

In addition, the tube 10 also defines a plurality of outlines 33. The outlines 33 are mutually identical. Furthermore, they are arranged in succession along the development axis 32a.

Thus, the outlines 33 are substantially formed along the development axis 32a by the inner surface 20 and determine the overall shape of the cavity.

In particular, preferably, the outline 33 is determined, also in this case, on a section plane 32b.

The outline 33 also defines a first extension area. The extension area is the portion of two-dimensional space contained within the outline 33.

Hence, the outline 33 is preferably inscribable in the circle determined on the section plane 32b.

The circle, in addition, itself defines a second extension area on the section plane 1b. Hence, the second area of extension being determined by the two-dimensional space contained within the circle, it can be as known obtained by the formula A=π*r². Advantageously, the outline 33 does not have a shape corresponding to the circle. In fact, advantageously, the first extension area is less than 90% of the second extension area. Even more in detail, preferably, the first extension area is less than 60% of the second extension area.

Thus, the outline 33 can be, even in the case of the tube 10, made according to different embodiments as described above for at least part of the polymer filaments 32, 40, 60.

In addition to what has been described, the tube 10 may also comprise an outer surface 40.

The outer surface 40 is also closed. Furthermore, the outer surface 40 develops around the inner surface 20. Then, the outer surface 40 wraps around the inner surface 20.

Preferably, moreover, the outer surface 40, which faces the outside of the tube 10 and therefore is not in contact with the cavity, is connected to the inner surface 20 via a wall 50.

The wall 50 is therefore surrounded by the surfaces 20, 40 and, therefore, the surfaces 20, 40 define opposite faces of the wall 50.

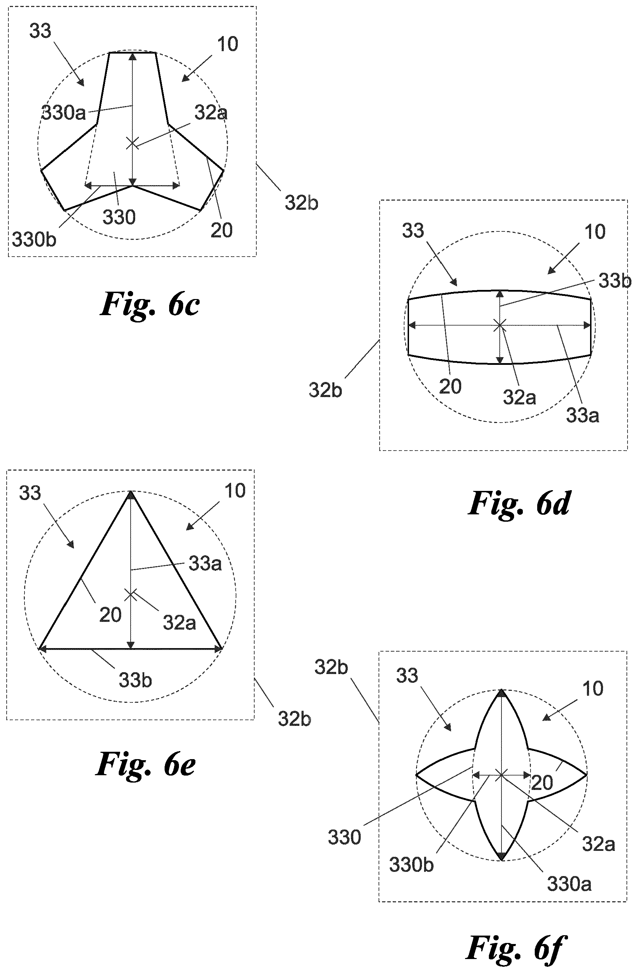

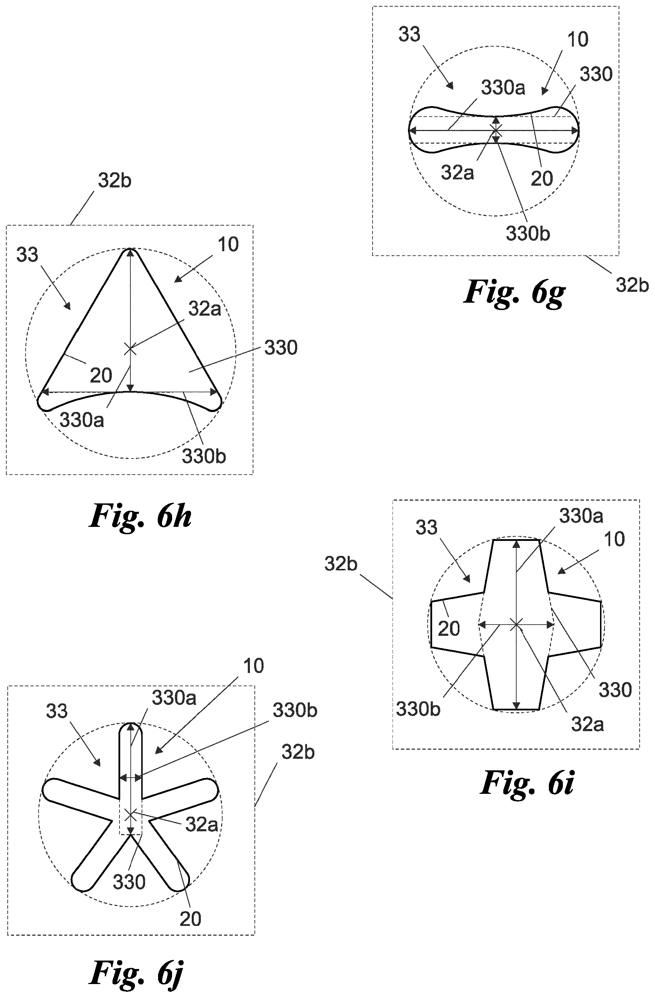

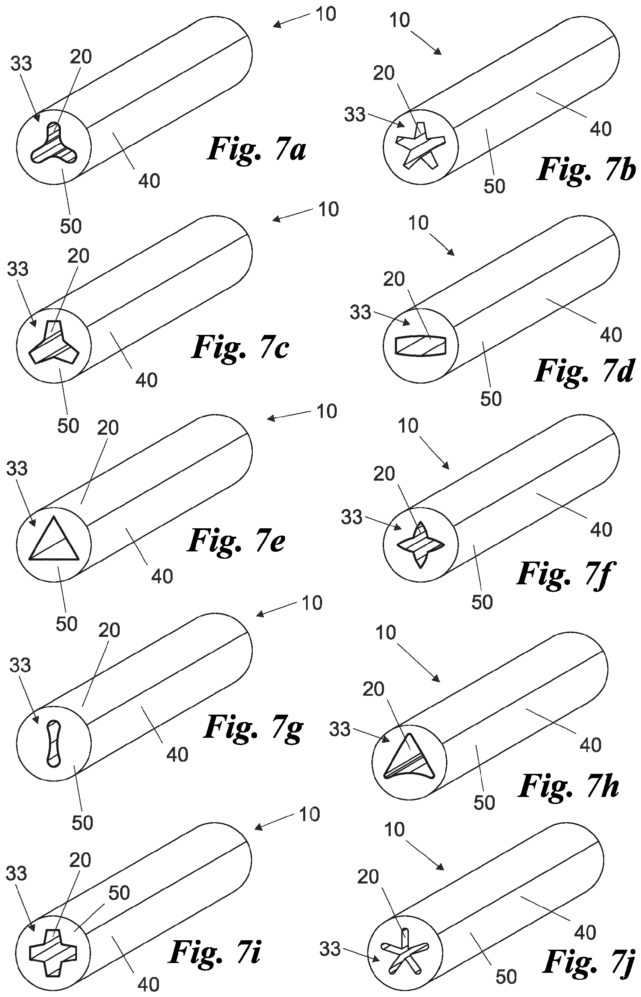

The outer surface 40 may thus be cylindrical, as shown in Figs. 6a-6j, 8a-8d and 9a-9d, or the outer surface 40 may alternatively be outlined as the inner surface 20. In this way, the surfaces 20, 40 determine a constant thickness for the wall 50, as shown for example in Figs. 5 and 7a-7d.

Of course, the tube 10 may be used with other tubes 10 to form a tube package for use in the first spinneret 1042.

Therefore, the plant 100 according to the invention also comprises a first spinneret 1042 comprising a plurality of tubes 10, forming a tube package, developing along a respective development axis 32a and of which at least part of these defines an outline 33 as described.

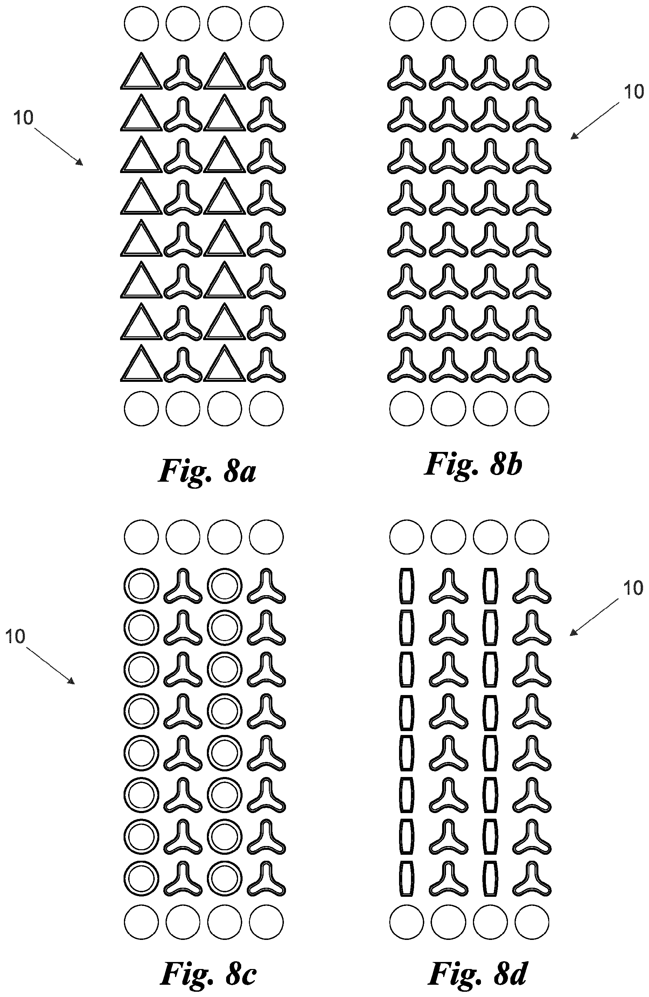

Thus, among the various embodiments, the package may comprise a plurality of tubes 10 all defining a same outline 33, as in Figs. 8a-8d.

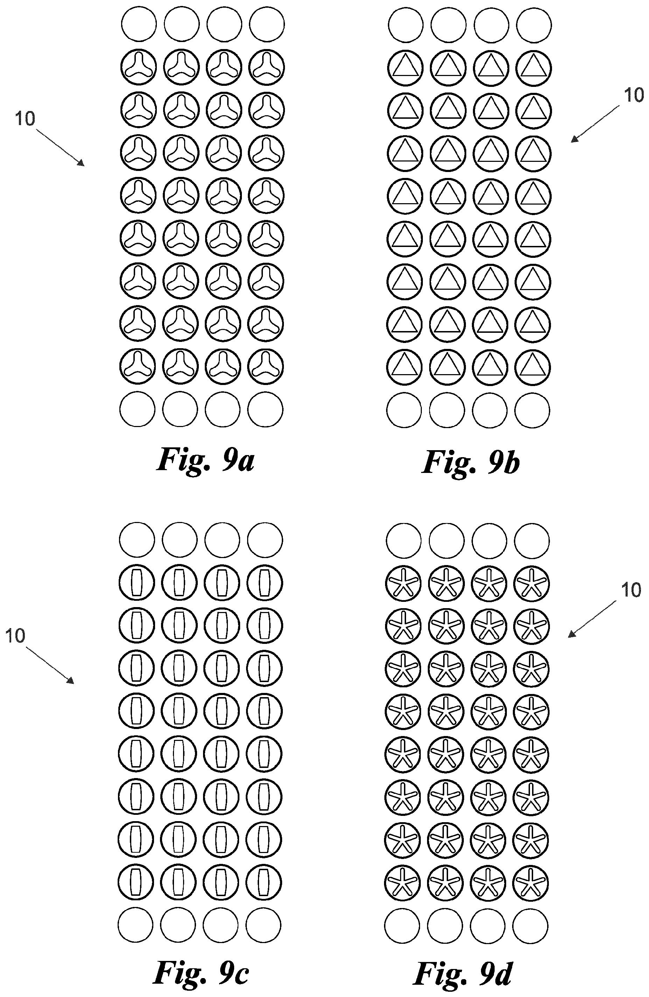

Or, the package may comprise a plurality of said tubes 10 defining respective mutually different outlines 33, as in Figs. 7a-7d and 9b-9d.

Or again, the package may comprise a plurality of tubes 10 and a plurality of tubes each defining a circular outline, i.e. having a conventional outline according to the known art, as in Figs. 7c and 9a.

Obviously, such tube packages 10 make it possible to obtain polymer filaments 32, 40, 60 with corresponding outlines 33 or circular outlines.

Of course, the invention also comprises a plant 100 including a first spinneret 1042 which, together with the first supply unit 1040, realizes a multi-row coaxial meltblown type device comprising a package as just described according to the different possible embodiments.

The plant 100 can, therefore, also comprise a calender 105.

The calender 105 is adapted to crush the tape 1 in such a way as to allow the penetration of the mixture 30 into the pores.

Of course, in order to manufacture the different layered tapes 1 as previously described in the different embodiments, the plant 100 may further comprise a second mixing unit 105.

Analogously to the first mixing unit 104, the second mixing unit 105 is adapted to manufacture the third layer 5 to allow its deposition on the matrix 2.

The second mixing unit 105 therefore preferably includes a second supply unit 1050 adapted to produce the particles 31, a second polymerization unit 1051 adapted to produce the first polymer filaments 32, and a second spinneret 1052 adapted to outline the first polymer filaments 32 before conveying the first polymer filaments 32 towards the particles 31 to mix them making a mixture 30 and comprising at least in part, in this regard, other tubes 10.

Alternatively or together with the second mixing unit 105, the plant 100 may comprise at least one second coaxial blowing unit 106.

If present, the second coaxial blowing unit 106 is adapted to allow the deposition of the fourth layer 6 on the matrix 2 and/or on the first layer 3 and/or on the third layer 5.

To increase the penetration of the mixture 30 into the matrix 2, one or more of the coaxial blowing units 102, 106 and mixing units 104, 105 may be provided with a suction unit 1011. If present, the suction unit 1011 is positioned on the opposite side of the support surface 101a so as to crush layers 3, 4, 5, 6 and matrix 2 towards the support surface 101a.

The invention also includes a novel method for manufacturing a composite tape, in particular the tape 1.

The method comprises at least depositing, in a first deposition step I the second layer 4 on a support surface 101a, depositing in a second deposition step II, the first layer 3 or the matrix 2 on the second layer 4, then depositing, in a third deposition step III, the matrix 2 on the first layer 3 or the first layer 3 on the matrix 2.

Then, the method comprises at least calendering the layers 3, 4 and the matrix 2 to manufacture the tape 1.

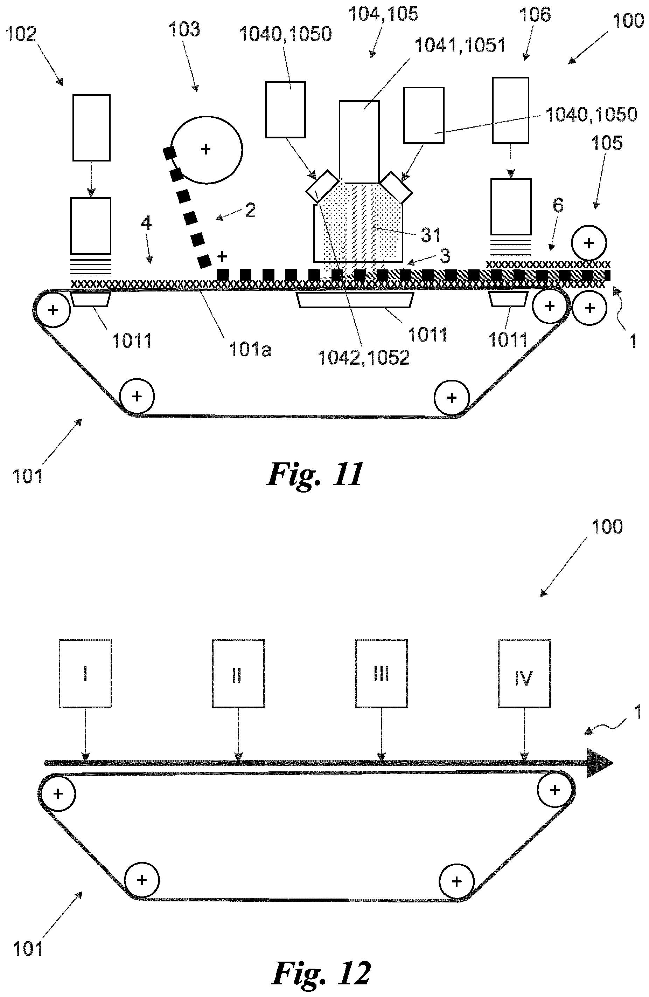

If the tape 1 also comprises the fourth layer 6, as shown in Figs. 12-13, preferably in the second deposition step II, the matrix 2 is deposited on the second layer 4, in the third deposition step III, the first layer 3 is deposited on the matrix 2, and in a fourth deposition step IV, the fourth layer 6 is deposited on the first layer 3.

The method may, therefore, comprise depositing, in a fifth deposition step V, a third layer 5 on the matrix 2.

Then, the fourth layer 6 may, in the fourth deposition step IV, be deposited on the third layer 5.

In this case, in the calendering step, all the layers 3, 4, 5, 6 and the matrix 2 are calendered to manufacture the tape 1.

The method may advantageously comprise that one or more of the layers 3, 4, 5, 6 and the matrix 2 is deposited on the support surface 101a inclined with respect to the support surface 101a so as to be neither perpendicular nor parallel to the support surface 101a. In this sense, it is for example intended that one or more of the polymer filaments 32, 40, 60, mixed or not mixed with the particles 31, and the polymeric fibres are conveyed towards the support surface 101a inclined with respect thereto.

In a preferred embodiment, only one or more of the layers 3, 5 and the matrix 2 are deposited inclined with respect to the support surface 101a.

Thus, the method may also provide that one or more of the layers 3, 4, 5, 6 and the matrix 2 is deposited on the support surface 101a perpendicularly thereto.

In a preferred embodiment, only the layers 4, 6 are deposited perpendicularly to the support surface 101a.

Further, the method may be performed in the order in which it was previously described and as shown in Figs. 12-13; or the method can be carried out by first providing for the second deposition step II, then a third deposition step III, then a first deposition step I.

For completion, the layers 4, 3 and the matrix 2 can then be tipped in a tipping station.

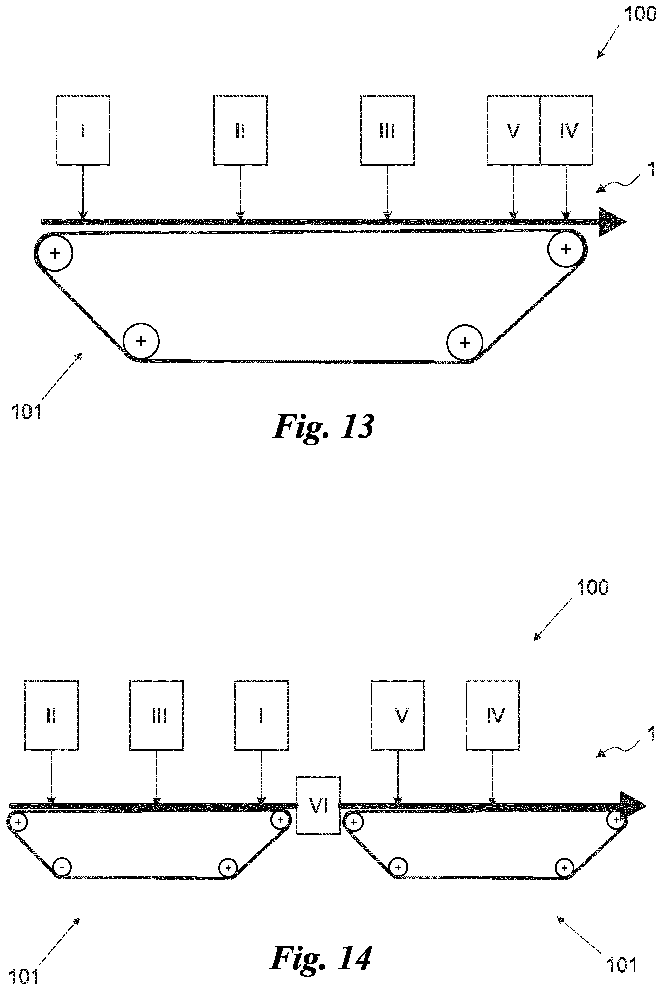

Then, the method may comprise inverting, in an overturning step VI, layers 4, 3 and matrix 2, before proceeding with a fifth deposition step V and with the fourth deposition step IV. This process is, in particular, shown in Fig. 14.

As already explained above, the tape 1 need not comprise the matrix 2.

In this case, in particular, the tape 1 could be used to manufacture wipes.

In this case, preferably, the tape 1 may essentially comprise a first layer 3.

The first layer 3 may, therefore, essentially include a plurality of polymer filaments 32 defining outlines 33 as previously described.

Or, the first layer 3 may comprise a plurality of cellulose particles 31 mixed with the first polymer filaments 32 so as to manufacture the mixture 30.

Then, the tape 1 may also include the second layer 4 in contact with the first layer 3.

In addition, the tape 1 may include the third layer 5 in contact with the first layer and/or the fourth layer 6 in contact with the third layer 5 at an opposite side of the second layer 4.

Similarly to the first layer 3, in this case, the third layer 5 may essentially include a plurality of polymer filaments 32 defining outlines 33 as previously described. Or, the third layer 5 may comprise a plurality of cellulose particles 31 mixed with the first polymer filaments 32 so as to manufacture the mixture 30.

To manufacture this type of tape 1, the plant 100 can be devoid of some elements including, for example, the unwinding unit 103.

In fact, preferably, the plant 100 that realizes this form of manufacturing the tape 1 includes the conveyor 101, the first coaxial blowing unit 102, the first polymerization unit 1041, the first spinneret 1042 and the calender 105.

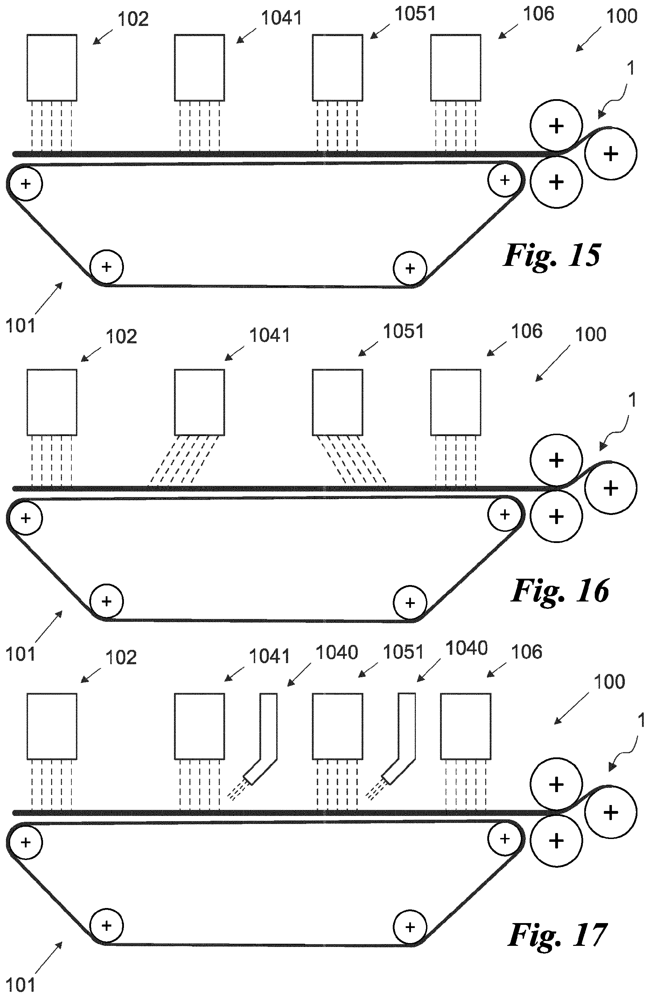

Of course, the plant 100 may also comprise, as shown in Figs. 15-16, the second polymerization unit 1051, the second spinneret 1052 and/or the second coaxial blowing unit 106.

The plant 100 could also include a first mixing unit 104 and/or a second mixing unit 105. The latter, if present, respectively include at least the first polymerization unit 1041 with the first spinneret 1042 and the second polymerization unit 1051 with the second spinneret 1052.

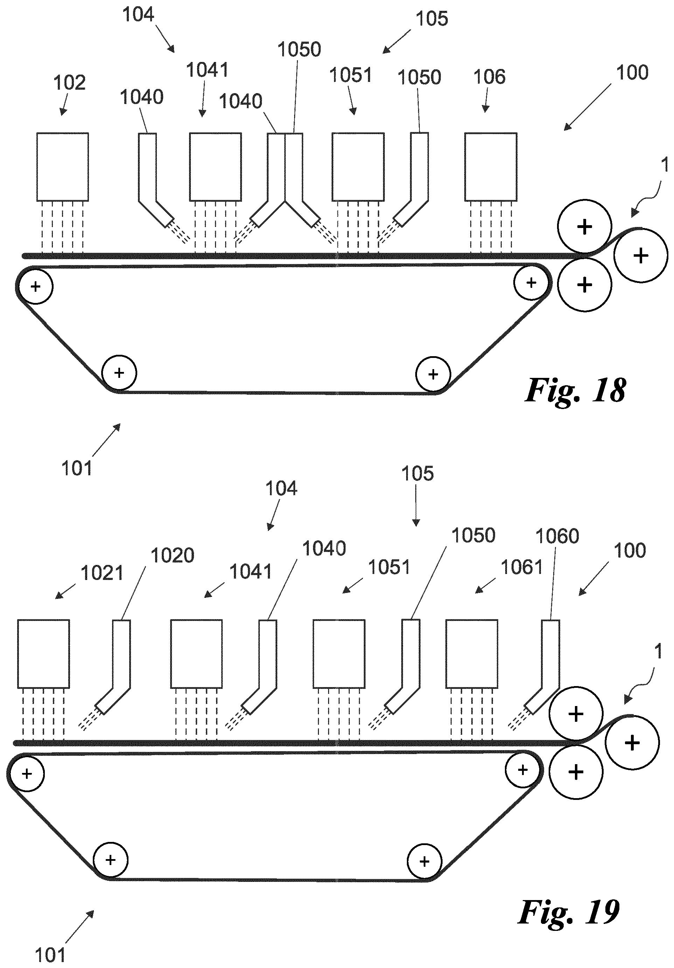

Further, the first mixing unit 104 and/or a second mixing unit 105 could also comprise, as shown in Figs. 17-19, respectively the first supply unit 1040 and/or the second supply unit 1040 adapted to produce particles 31.

Then, and one or more of the spinnerets 1042, 1052 could, in this embodiment, be adapted to outline the first polymer filaments 32 before conveying the first polymer filaments 32 towards the particles 31 to mix them so as to manufacture the mixture 30.

In this regard, also one or more of the first coaxial blowing unit 102 and the second blowing unit 106 could respectively comprise a third supply unit 1020 and/or a fourth supply unit 1060.

Then, one or more of the first coaxial blowing unit 102 and the second blowing unit 106 may comprise a third polymerization unit 1021 having a third spinneret and a fourth polymerization unit 1061 having a fourth spinneret.

Third and fourth polymerization units 1021, 1061 are preferably analogous to first and second polymerization units 1041, 1051 as well as third and fourth spinneret are preferably analogous to first and second spinneret 1042, 1052.

The method for manufacturing the tape 1, in this embodiment, may also be different from that previously described.

In fact, in this case, the method preferably comprises a first deposition step I similar to the previous one followed by a second deposition step II in which the first layer 3 is deposited on the second layer 4.

Then, the method comprises a calendering step in which first and second layers 3, 4 are calendered to manufacture the tape 1.

In addition, the methods could also comprise a fifth deposition step V in which the third layer 5 is deposited on the first layer 3 and a fourth deposition step IV in which the fourth layer 6 is deposited on the third layer 5.

In this case, the calendering is carried out on all layers 3, 4, 5, 6 to manufacture the tape 1.

This method may also include, as shown in Fig. 16, that one or more layers 3, 4, 5, 6 are deposited on the support surface 101a inclined with respect to the support surface 101a so as to be neither perpendicular nor parallel to the support surface 101a. Also in this case, it is for example intended that one or more of the polymer filaments 32, 40, 60, mixed or not mixed with the particles 31, and the polymeric fibres are conveyed towards the support surface 101a inclined with respect thereto. Further, one or more of the layers 3, 4, 5, 6 may be deposited on the support surface 101a perpendicularly thereto.

The composite tape 1, and related plants and manufacturing method, according to the invention achieve important advantages.

In fact, the composite tape 1, and related plant and manufacturing method, are economically feasible.

Furthermore, the composite tape 1, and related plant and manufacturing method, make it possible to obtain a high production volume with a less expensive production unit and raw materials, in particular without adhesives.

Indeed, a particular advantage of the method according to the present invention is that it exploits the adhesiveness of the filaments formed in situ to ensure the integrity of the structure and the containment of the particles without the need to add further glue.

Furthermore, the process allows very high production volumes for the resulting continuous absorbent composite tape, as it can be used at very high production speeds, higher than about 500 m/min, or at about 700 m/min, or even at about 1000 m/min.

In addition, the width of the tape can be greater than about 1 m, or 3 m, or 5 m, or 7 m, so as to achieve overall production rates of greater than 500 m²/min, or 1000 m²/min, or 2000 m²/min, or 4000 m²/min, or even 5000 m²/min.

In conclusion, the composite tape 1 is, in view of the advantages just described, very efficient in that it does not compromise the containment of the particles before and during production, but also during use.

The invention can be modified to create different versions falling within the scope of the inventive concept defined by the claims.

In this context, all the details can be replaced by equivalent elements and any materials, shapes and dimensions can be used.

## Claims

1. Composite tape (1) comprising:
- one or more matrices (2) of high thickness polymeric fibres comprising pores;
- at least one first layer (3) in contact with said matrix (2) and including a mixture (30) at least in part penetrating said pores and comprising mutually mixed together:
- a plurality of particles (31) of cellulose, and
- a plurality of first polymer filaments (32) defining, before being mixed with said particles (31), a development axis (32a) and defining at least in part a plurality of mutually identical shapes (33) arranged in succession along said development axis (32a);
- a second layer (4) of second polymer filaments (40) forming a surface layer of said tape (1) and placed in contact with said matrix (2) and/or said first layer (3) without penetrating said pores;
and **characterized in that**
- said outline (33) is determined on a section plane (32b) normal to said development axis (32a), defining a first extension area on said section plane (32b) and being inscribable in a circle determined on said section plane (32b) and defining a second extension area on said section plane (32b);
- said first extension area being less than 90% of said second extension area.

2. Tape (1) according to Claim 1, wherein said first extension area is less than 60% of said second extension area.

3. Tape (1) according to any one of the preceding claims, wherein said outline (33) is convex and defines at least a first maximum dimension (33a) and a second maximum dimension (33b) perpendicular to said first dimension (33a) and less than 90% of said first maximum dimension (33a).

4. Tape (1) according to the preceding claim, wherein said second dimension (33b) is less than 60% of said first dimension (33a).

5. Tape (1) according to any one of Claims 1-2, wherein said outline (33) is concave and includes at least one convex portion (330) identifiable within said outline (33) in such a way as to be delimited by at least part of said outline (33) and defining at least a third maximum dimension (330a) and a fourth maximum dimension (330b), perpendicular to said third dimension (330a) and less than 90% of said third maximum dimension (330a).

6. Tape (1) according to the preceding claim, wherein said fourth dimension (330b) is less than 60% of said third dimension (330a).

7. Tape (1) according to any one of Claims 6-7, wherein said outline (33) is formed by two or more said mutually crossed convex portions (330).

8. Tape (1) according to any one of the preceding claims, further comprising:
- a third layer (5) in contact with said matrix (2) at a side opposite to said first layer (3) and also including a said mixture (30) at least partly penetrating said pores, and/or
- a fourth layer (6) of continuous third polymer filaments (60) forming a surface layer of said tape (1) and placed in contact with said matrix (2) and/or said first layer (3) and/or said third layer (5) without penetrating said pores at a side opposite to said second layer (4).

9. Tape (1) according to any one of the preceding claims, wherein at least part of said second polymer filaments (40) and/or said third polymer filaments (60) also define a respective said development axis (32a) and respective said outlines (33).

10. Plant (100) for manufacturing a tape (1) according to any one of the preceding claims, comprising at least:
- a conveyor (101) defining a support surface (101a) on which to manufacture said tape (1),
- at least one first coaxial blowing unit (102) adapted to allow the deposition of said second layer (4) on said conveyor (101),
- at least one unwinding unit (103) adapted to allow the deposition of said matrix (2) on said second layer (4);
- a first mixing unit (104) adapted to manufacture said first layer (3) to allow its deposition on said matrix (2) and including:
- a first supply unit (1040) adapted to produce said particles (31),
- a first polymerization unit (1041) adapted to produce said first polymer filaments (32), and
- a first spinneret (1042) adapted to outline said first polymer filaments (32) before conveying said first polymer filaments (32) towards said particles (31) to mix them to make a said mixture (30); and
- a calender (105) adapted to crush said tape (1) in such a way as to allow the penetration of said mixture (30) into said pores;
and **characterized in that**
- said first spinneret (1042) comprises a plurality of tubes (10) developing along a respective said development axis (32a) and of which at least part of these defines a said outline (33) determined on said section plane (32b).

11. Plant (100) according to the preceding claim, further comprising:
- a second mixing unit (105) adapted to manufacture said third layer (5) to allow its deposition on said matrix (2) and including:
- a second supply unit (1050) adapted to produce said particles (31),
- a second polymerization unit (1051) adapted to produce said first polymer filaments (32), and
- a second spinneret (1052) adapted to outline said first polymer filaments (32) before conveying said first polymer filaments (32) towards said particles (31) to mix them to manufacture a said mixture (30) and comprising other said tubes (10); and/or
- at least one second coaxial blowing unit (106) adapted to allow the deposition of said fourth layer (6) on said matrix (2) and/or said first layer (3) and/or said third layer (5).

12. Method for manufacturing a tape (1) according to any one of the preceding claims 1-9, comprising:
- depositing (I) said second layer (4) on a support surface (101a);
- depositing (II) said first layer (3) or said matrix (2) on said second layer (4);
- depositing (III) said matrix (2) on said first layer (3) or said first layer (3) on said matrix (2);
- calendering at least said layers (3, 4) and said matrix (2) to manufacture said tape (1).

13. Method according to the preceding claim, comprising:
- depositing (V) a third layer (5) on said matrix (2);
- depositing (IV) a fourth layer (6) on said third layer (5);
- calendering said layers (3, 4, 5, 6) and said matrix (2) to manufacture said tape (1).

14. Method according to any one of Claims 12-13, wherein one or more of said layers (3, 4, 5, 6) and said matrix (2) is deposited on said support surface (101a) inclined with respect to said support surface (101a) so as to be neither perpendicular nor parallel to said support surface (101a).

15. Method according to any one of Claims 12-14, wherein one or more of said layers (3, 4, 5, 6) and said matrix (2) is deposited on said support surface (101a) perpendicularly to said support surface (101a).

## Patentansprüche

1. Verbundband (1), umfassend:
- eine oder mehrere Matrizen (2) aus hochdicken Polymerfasern, die Poren umfassen;
- mindestens eine erste Schicht (3), die mit der Matrix (2) in Kontakt steht und enthaltend eine Mischung (30), die mindestens teilweise in die Poren eindringt und umfassend:
- eine Mehrzahl von Cellulosen Partikeln (31), und
- eine Mehrzahl aus ersten Polymerfilamenten (32), die vor dem Mischen mit den Partikeln (31) eine Entwicklungsachse (32a) definieren und mindestens teilweise eine Mehrzahl von gegenseitig identischen Formen (33) definieren, die entlang der Entwicklungsachse (32a) hintereinander angeordnet sind,
die gegenseitig zusammen gemischt werden;
- eine zweite Schicht (4) aus zweiten Polymerfilamenten (40), die eine Oberflächenschicht des Bandes (1) bildet und in Kontakt mit der Matrix (2) und/oder der ersten Schicht (3) angeordnet ist, ohne in die Poren einzudringen; und **dadurch gekennzeichnet, dass**
- die Kontur (33) auf einer Schnittebene (32b) bestimmt wird, die normal zu der Entwicklungsachse (32a) ist, wobei sie einen ersten Erstreckungsbereich auf der Schnittebene (32b) definiert und in einen auf der Schnittebene (32b) bestimmten Kreis einschreibbar ist und einen zweiten Erstreckungsbereich auf der Schnittebene (32b) definiert;
- der erste Erstreckungsbereich kleiner als 90 % des zweiten Erstreckungsbereiches ist.

2. Band (1) nach Anspruch 1, wobei der erste Erstreckungsbereich kleiner als 60 % des zweiten Erstreckungsbereiches ist.

3. Band (1) nach einem der vorhergehenden Ansprüche, wobei die Kontur (33) konvex ausgebildet ist und mindestens eine erste maximale Abmessung (33a) und eine zweite maximale Abmessung (33b) definiert, die senkrecht zu der ersten Abmessung (33a) ist und kleiner als 90 % der ersten maximalen Abmessung (33a) ist.

4. Band (1) nach dem vorhergehenden Anspruch, wobei die zweite Abmessung (33b) kleiner als 60 % der ersten Abmessung (33a) ist.

5. **.** Band (1) nach einem der Ansprüche 1-2, wobei die Kontur (33) konkav ausgebildet ist und mindestens einen konvexen Abschnitt (330) umfasst, der innerhalb der Kontur (33) identifiziert werden kann, um durch mindestens einen Teil der Kontur (33) begrenzt zu werden und mindestens eine dritte maximale Abmessung (330a) und eine vierte maximale Abmessung (330b) definiert, die senkrecht zu der dritten Abmessung (330a) ist und kleiner als 90 % der dritten maximalen Abmessung (330a) ist.

6. **.** Band (1) nach dem vorhergehenden Anspruch, wobei die vierte Abmessung (330b) kleiner als 60 % der dritten Abmessung (330a) ist.

7. **.** Band (1) nach einem der Ansprüche 6-7, wobei die Kontur (33) durch zwei oder mehr der gegenseitig gekreuzten konvexen Abschnitte (330) gebildet wird.

8. **.** Band (1) nach einem der vorhergehenden Ansprüche, ferner umfassend:
- eine dritte Schicht (5), die mit der Matrix (2) an einer der ersten Schicht (3) gegenüberliegenden Seite in Kontakt steht und ebenfalls enthaltend die Mischung (30), die mindestens teilweise in die Poren eindringt, und/oder
- eine vierte Schicht (6) aus kontinuierlichen dritten Polymerfilamenten (60), die eine Oberflächenschicht des Bandes (1) bildet und in Kontakt mit der Matrix (2) und/oder der ersten Schicht (3) und/oder der dritten Schicht (5) angeordnet ist, ohne in die Poren an einer der zweiten Schicht (4) gegenüberliegenden Seite einzudringen.

9. **.** Band (1) nach einem der vorhergehenden Ansprüche, wobei mindestens ein Teil der zweiten Polymerfilamente (40) und/oder der dritten Polymerfilamente (60) ebenfalls jeweils die Entwicklungsachse (32a) und die jeweiligen Konturen (33) definieren.

10. **.** Anlage (100) zur Herstellung eines Bandes (1) nach einem der vorhergehenden Ansprüche, mindestens umfassend:
- ein Förderband (101), das eine Stützoberfläche (101a) definiert, auf der das Band (1) hergestellt wird,
- mindestens eine erste koaxiale Blaseinheit (102), die eingerichtet ist, das Aufbringen der zweiten Schicht (4) auf das Förderband (101) zu ermöglichen,
- mindestens eine Abwicklungseinheit (103), die eingerichtet ist, das Aufbringen der Matrix (2) auf die zweite Schicht (4) zu ermöglichen;
- eine erste Mischeinheit (104), die eingerichtet ist, die erste Schicht (3) herzustellen, um deren Aufbringen auf die Matrix (2) zu ermöglichen, und umfassend:
- eine erste Versorgungseinheit (1040), die eingerichtet ist, die Partikel (31) herzustellen,
- eine erste Polymerisationseinheit (1041), die eingerichtet ist, die ersten Polymerfilamente (32) herzustellen, und
eine erste Spinndüse (1042), die eingerichtet ist, die ersten Polymerfilamente (32) zu umreißen, bevor die ersten Polymerfilamente (32) zu den Partikeln (31) gefördert werden, um sie zu mischen, um die Mischung (30) herzustellen; und
- eine Aufwickelwalze (105), die dazu eingerichtet ist, das Band (1) zu pressen, sodass das Eindringen der Mischung (30) in die Poren ermöglicht wird;
und **dadurch gekennzeichnet, dass**
- die erste Spinndüse (1042) eine Mehrzahl von Rohren (10) umfasst, die sich entlang der jeweiligen Entwicklungsachse (32a) erstrecken und von denen mindestens ein Teil die Kontur (33) definiert, die auf der Schnittebene (32b) bestimmt wird.

11. **.** Anlage (100) nach dem vorhergehenden Anspruch, ferner umfassend:
- eine zweite Mischeinheit (105), die eingerichtet ist, die dritte Schicht (5) herzustellen, um deren Aufbringen auf die Matrix (2) zu ermöglichen, und umfassend:
- eine zweite Versorgungseinheit (1050), die eingerichtet ist, die Partikel (31) zu herzustellen,
- eine zweite Polymerisationseinheit (1051), die eingerichtet ist, die ersten Polymerfilamente (32) herzustellen, und
- eine zweite Spinndüse (1052), die eingerichtet ist, die ersten Polymerfilamente (32) zu umreißen, bevor die ersten Polymerfilamente (32) zu den Partikeln (31) gefördert werden, um sie zu mischen und die Mischung (30) herzustellen, und umfassend weitere Rohre (10); und/oder
- mindestens eine zweite koaxiale Blaseinheit (106), die eingerichtet ist, das Aufbringen der vierten Schicht (6) auf die Matrix (2) und/oder der ersten Schicht (3) und/oder der dritten Schicht (5) zu ermöglichen.

12. **.** Verfahren zur Herstellung eines Bandes (1) nach einem der Ansprüche 1-9, umfassend:
- Aufbringen (I) der zweiten Schicht (4) auf eine Stützoberfläche (101a);
- Aufbringen (II) der ersten Schicht (3) oder der Matrix (2) auf die zweite Schicht (4);
- Aufbringen (III) der Matrix (2) auf die erste Schicht (3) oder der ersten Schicht (3) auf die Matrix (2);
- Kalandrieren mindestens der Schichten (3, 4) und der Matrix (2), um das Band (1) herzustellen.

13. **.** Verfahren nach dem vorhergehenden Anspruch, umfassend:
- Aufbringen (V) einer dritten Schicht (5) auf die Matrix (2);
- Aufbringen (IV) einer vierten Schicht (6) auf die dritte Schicht (5);
- Kalandrieren der Schichten (3, 4, 5, 6) und der Matrix (2), um das Band (1) herzustellen.

14. **.** Verfahren nach einem der Ansprüche 12-13, wobei eine oder mehrere der Schichten (3, 4, 5, 6) und die Matrix (2) auf der Stützoberfläche (101a) geneigt in Bezug auf die Stützoberfläche (101a) aufgebracht werden, so dass sie weder senkrecht noch parallel zu der Stützoberfläche (101a) sind.

15. **.** Verfahren nach einem der Ansprüche 12-14, wobei eine oder mehrere der Schichten (3, 4, 5, 6) und die Matrix (2) senkrecht zu der Stützoberfläche (101a) auf der Stützoberfläche (101a) aufgebracht werden.

## Revendications

1. Bande composite (1) comprenant :
- une ou plusieurs matrices (2) de fibres polymériques de haute épaisseur comprenant des pores ;
- au moins une première couche (3) en contact avec ladite matrice (2) et comprenant un mélange (30) pénétrant au moins partiellement lesdits pores et comprenant mutuellement mélangés ensemble :
- une pluralité de particules (31) de cellulose, et
- une pluralité de premiers filaments polymériques (32) définissant, avant d'être mélangés avec lesdites particules (31), un axe de développement (32a) et définissant au moins en partie une pluralité de formes mutuellement identiques (33) disposées successivement le long dudit axe de développement (32a) ;
- une seconde couche (4) de seconds filaments polymériques (40) formant une couche de surface de ladite bande (1) et placée en contact avec ladite matrice (2) et/ou ladite première couche (3) sans pénétrer lesdits pores ;
et **caractérisée en ce que**
- ledit contour (33) est déterminé sur un plan de section (32b) normal audit axe de développement (32a), définissant une première zone d'extension sur ledit plan de section (32b) et étant inscriptible dans un cercle déterminé sur ledit plan de section (32b) et définissant une seconde zone d'extension sur ledit plan de section (32b) ;
- ladite première zone d'extension étant inférieure à 90 % de ladite seconde zone d'extension.

2. Bande (1) selon la Revendication 1, dans laquelle ladite première zone d'extension est inférieure à 60 % de ladite seconde zone d'extension.

3. Bande (1) selon l'une quelconque des revendications précédentes, dans laquelle ledit contour (33) est convexe et définit au moins une première dimension maximale (33a) et une seconde dimension maximale (33b) perpendiculaire à ladite première dimension (33a) et inférieure à 90 % de ladite première dimension maximale (33a).

4. Bande (1) selon la revendication précédente, dans laquelle ladite seconde dimension (33b) est inférieure à 60 % de ladite première dimension (33a).

5. Bande (1) selon l'une quelconque des Revendications 1-2, dans laquelle ledit contour (33) est concave et comprend au moins une portion convexe (330) identifiable à l'intérieur dudit contour (33) de manière à être délimitée par au moins une partie dudit contour (33) et définissant au moins une troisième dimension maximale (330a) et une quatrième dimension maximale (330b), perpendiculaire à ladite troisième dimension (330a) et inférieure à 90 % de ladite troisième dimension maximale (330a).

6. Bande (1) selon la revendication précédente, dans laquelle ladite quatrième dimension (330b) est inférieure à 60 % de ladite troisième dimension (330a).

7. Bande (1) selon l'une quelconque des Revendications 6-7, dans laquelle ledit contour (33) est formé par deux ou plusieurs desdites portions convexes croisées mutuellement (330).

8. Bande (1) selon l'une quelconque des revendications précédentes, comprenant en outre :
- une troisième couche (5) en contact avec ladite matrice (2) sur un côté opposé à ladite première couche (3) et comprenant également ledit mélange (30) pénétrant au moins partiellement lesdits pores, et/ou
- une quatrième couche (6) de troisièmes filaments polymériques continus (60) formant une couche de surface de ladite bande (1) et placée en contact avec ladite matrice (2) et/ou ladite première couche (3) et/ou ladite troisième couche (5) sans pénétrer lesdits pores sur un côté opposé à ladite seconde couche (4).

9. Bande (1) selon l'une quelconque des revendications précédentes, dans laquelle au moins une partie desdits seconds filaments polymériques (40) et/ou desdits troisièmes filaments polymériques (60) définissent également respectivement ledit axe de développement (32a) et lesdits contours respectifs (33).

10. Installation (100) pour fabriquer une bande (1) selon l'une quelconque des revendications précédentes, comprenant au moins :
- un convoyeur (101) définissant une surface de support (101a) sur laquelle fabriquer ladite bande (1),
- au moins une première unité de soufflage coaxiale (102) adaptée pour permettre le dépôt de ladite seconde couche (4) sur ledit convoyeur (101),
- au moins une unité de déroulement (103) adaptée pour permettre le dépôt de ladite matrice (2) sur ladite seconde couche (4) ;
- une première unité de mélange (104) adaptée pour fabriquer ladite première couche (3) afin de permettre son dépôt sur ladite matrice (2) et comprenant :
- une première unité d'alimentation (1040) adaptée pour produire lesdites particules (31),
- une première unité de polymérisation (1041) adaptée pour produire lesdits premiers filaments polymériques (32), et
- une première filière (1042) adaptée pour profiler lesdits premiers filaments polymériques (32) avant de convoyer lesdits premiers filaments polymériques (32) vers lesdites particules (31) afin de les mélanger pour réaliser ledit mélange (30) ; et
- une calandre (105) adaptée pour écraser ladite bande (1) de manière à permettre la pénétration dudit mélange (30) dans lesdits pores ;
et **caractérisée en ce que**
- ladite première filière (1042) comprend une pluralité de tubes (10) se développant le long dudit axe de développement respectif (32a) et dont au moins une partie de ceux-ci définit ledit contour (33) déterminé sur ledit plan de section (32b).

11. Installation (100) selon la revendication précédente, comprenant en outre :
- une seconde unité de mélange (105) adaptée pour fabriquer ladite troisième couche (5) afin de permettre son dépôt sur ladite matrice (2) et comprenant :
- une seconde unité d'alimentation (1050) adaptée pour produire lesdites particules (31),
- une seconde unité de polymérisation (1051) adaptée pour produire lesdits premiers filaments polymériques (32), et
- - une seconde filière (1052) adaptée pour profiler lesdits premiers filaments polymériques (32) avant de convoyer lesdits premiers filaments polymériques (32) vers lesdites particules (31) afin de les mélanger pour fabriquer ledit mélange (30) et comprenant d'autres desdits tubes (10) ; et/ou
- au moins une seconde unité de soufflage coaxiale (106) adaptée pour permettre le dépôt de ladite quatrième couche (6) sur ladite matrice (2) et/ou ladite première couche (3) et/ou ladite troisième couche (5).

12. Procédé pour fabriquer une bande (1) selon l'une quelconque des revendications 1-9, comprenant :
- déposer (I) ladite seconde couche (4) sur une surface de support (101a) ;
- déposer (II) ladite première couche (3) ou ladite matrice (2) sur ladite seconde couche (4) ;
- déposer (III) ladite matrice (2) sur ladite première couche (3) ou ladite première couche (3) sur ladite matrice (2) ;
- calandrer au moins lesdites couches (3, 4) et ladite matrice (2) pour fabriquer ladite bande (1).

13. Procédé selon la revendication précédente, comprenant :
- déposer (V) une troisième couche (5) sur ladite matrice (2) ;
- déposer (IV) une quatrième couche (6) sur ladite troisième couche (5) ;
- calandrer lesdites couches (3, 4, 5, 6) et ladite matrice (2) pour fabriquer ladite bande (1).

14. Procédé selon l'une quelconque des Revendications 12-13, dans lequel une ou plusieurs desdites couches (3, 4, 5, 6) et ladite matrice (2) sont déposées sur ladite surface de support (101a) inclinées par rapport à ladite surface de support (101a) de manière à n'être ni perpendiculaires ni parallèles à ladite surface de support (101a).

15. Procédé selon l'une quelconque des Revendications 12-14, dans lequel une ou plusieurs desdites couches (3, 4, 5, 6) et ladite matrice (2) sont déposées sur ladite surface de support (101a) perpendiculairement à ladite surface de support (101a).
